# EUROPEAN PATENT APPLICATION

(11) **EP 2 284 257 A1**
(43) Date of publication of application: **16.02.2011**
(21) Application number: 10185629.2
(22) Date of filing: 18.01.2006
(51) Int. Cl.: C12N 9/10, A61K 39/04

(54) **Mycobacteria expressing a cap-specific mannosyl transferase**

(30) Priority: 18.01.2005 US 644308 P
(62) Divisional of application: 06700813.6
(71) Applicant: Vereniging voor Christelijk Hoger Onderwijs, Wetenschappelijk Onderzoek en Patiëntenzorg, 1081 HV Amsterdam (NL)
(72) Inventor: Appelmelk, Bernard Jan, 1071 MT Amsterdam (NL); Bitter, Wilhelmus, 3992 XS Houten (NL); Van der ley, Peter André, 3572 DJ Utrecht (NL)
(74) Representative: Swinkels, Bart Willem

(57) **Abstract**

The present invention relates to mycobacterial lipoarabinomannan cap-specific mannosyl transferases and nucleic acid encoding such transferases. The invention further relates to *Mycobacteria* in which the lipoarabinomannan cap-specific mannosyl transferases are expressed, and to the use of such *Mycobacteria* used as vaccines against mycobacterial diseases.

SEQUENCE LISTING

<110>  Vereniging voor Christelijk hoger Onderwijs,
       Wetenschappelijk Onderzoek en Patientenzorg

<120>  Mycobacteria expressing a cap-specific mannosyl transferase

<130>  P6000254EP1

<150>  US 60/644,308
<151>  2005-01-18

<160>  8

<170>  PatentIn version 3.3

<210>  1
<211>  556
<212>  PRT
<213>  Mycobacterium tuberculosis

<400>  1

Met His Ala Ser Arg Pro Gly Ala Pro Pro His Ala Gly Leu Pro Ser
1               5                   10                  15

Arg Arg Thr Ala Gly Asp Gln Asp His Arg Ala Asp Pro Lys Val Thr
            20                  25                  30

Arg Ile Met Ser Ala Ser Thr Leu Glu Gln Pro Ala Ala Ala His Val
        35                  40                  45

Asp Glu Leu Val Ala Arg Met Arg Gly Arg Leu Leu Asp Pro Leu Ala
    50                  55                  60

Ile Ala Val Leu Ala Ala Val Ile Ser Gly Ala Trp Ala Ser Arg Pro
65                  70                  75                  80

Ser Leu Trp Phe Asp Glu Gly Ala Thr Ile Ser Ala Ser Ala Ser Arg
                85                  90                  95

Thr Leu Pro Glu Leu Trp Ser Leu Leu Gly His Ile Asp Ala Val His
            100                 105                 110

Gly Leu Tyr Tyr Leu Leu Met His Gly Trp Phe Ala Ile Phe Pro Pro
        115                 120                 125

Thr Glu Leu Trp Ser Arg Leu Pro Ser Cys Leu Ala Ile Gly Ala Ala
    130                 135                 140

Ala Ala Gly Val Val Val Phe Ala Lys Gln Phe Ser Gly Arg Thr Thr
145                 150                 155                 160

Ala Val Cys Ala Gly Ala Val Phe Ala Ile Leu Pro Arg Val Thr Trp
                165                 170                 175

Ala Gly Ile Glu Ala Arg Ser Ser Ala Leu Ser Val Ala Ala Ala Val
            180                 185                 190

Trp Leu Thr Val Leu Leu Val Ala Ala Val Arg Cys Asn Thr Gln Arg
        195                 200                 205

Arg Trp Leu Leu Tyr Ala Leu Val Leu Met Leu Ser Ile Leu Val Ser
    210                 215                 220

Ile Asn Leu Ala Leu Leu Val Pro Ala Tyr Ala Thr Met Val Pro Leu
225                 230                 235                 240

Leu Ala Ser Gly Lys Ser Arg Lys Ser Pro Val Ile Trp Trp Thr Val
                245                 250                 255

Val Thr Ala Ala Ala Leu Gly Ala Met Thr Pro Phe Ile Leu Phe Ala
            260                 265                 270

His Gly Gln Val Trp Gln Val Gly Trp Ile Ala Gly Leu Asn Arg Asn
        275                 280                 285

Ile Ile Leu Asp Val Ile His Arg Gln Tyr Phe Asp His Ser Val Pro
    290                 295                 300

Phe Ala Ile Leu Ala Gly Leu Ile Val Ala Ala Gly Ile Ala Ala His
305                 310                 315                 320

Leu Ala Gly Ala Arg Gly Pro Gly Gly Asp Thr His Arg Leu Val Leu
                325                 330                 335

Val Ser Ala Ala Trp Ile Val Val Pro Thr Ala Val Val Leu Ile Tyr
            340                 345                 350

Ser Ala Thr Val Glu Pro Ile Tyr Tyr Pro Arg Tyr Leu Ile Leu Thr
        355                 360                 365

Ala Pro Ala Ala Ala Val Ile Leu Ala Val Cys Val Val Thr Ile Ala
    370                 375                 380

Arg Lys Pro Trp Leu Ile Ala Gly Val Val Phe Leu Leu Ala Ala Ala
385                 390                 395                 400

Ala Phe Pro Asn Tyr Phe Phe Thr Gln Arg Gly Pro Tyr Ala Lys Glu
                405                 410                 415

Gly Trp Asp Tyr Ser Gln Val Ala Asp Val Ile Ser Ala His Ala Lys
            420                 425                 430

Pro Gly Asp Cys Leu Leu Val Asp Asn Thr Ala Gly Trp Arg Pro Gly
        435                 440                 445

Pro Ile Arg Ala Leu Leu Ala Thr Arg Pro Ala Ala Phe Arg Ser Leu
    450                 455                 460

Ile Asp Val Glu Arg Gly Thr Tyr Gly Pro Lys Val Gly Thr Leu Trp
465                 470                 475                 480

Asp Gly His Val Ala Val Trp Leu Thr Thr Ala Lys Ile Asp Lys Cys
                485                 490                 495

Pro Thr Leu Trp Thr Ile Ala Asn Arg Asp Lys Ser Leu Pro Asp His
            500                 505                 510

Gln Val Gly Glu Met Leu Ser Pro Gly Thr Gly Phe Gly Arg Thr Pro
        515                 520                 525

Val Tyr Arg Phe Pro Ser Tyr Leu Gly Phe Arg Ile Val Glu Arg Trp
    530                 535                 540

Gln Phe His Tyr Ser Gln Val Val Lys Ser Thr Arg
545                 550                 555

<210>  2
<211>  565
<212>  PRT
<213>  Mycobacterium tuberculosis

<400>  2

Met Leu Leu Cys Lys Ala Phe Trp Glu Met His Ala Ser Arg Pro Gly
1               5                   10                  15

Ala Pro Pro His Ala Gly Leu Pro Ser Arg Arg Thr Ala Gly Asp Gln
            20                  25                  30

Asp His Arg Ala Asp Pro Lys Val Thr Arg Ile Met Ser Ala Ser Thr
        35                  40                  45

Leu Glu Gln Pro Ala Ala Ala His Val Asp Glu Leu Val Ala Arg Met
    50                  55                  60

Arg Gly Arg Leu Leu Asp Pro Leu Ala Ile Ala Val Leu Ala Ala Val
65                  70                  75                  80

Ile Ser Gly Ala Trp Ala Ser Arg Pro Ser Leu Trp Phe Asp Glu Gly
                85                  90                  95

Ala Thr Ile Ser Ala Ser Ala Ser Arg Thr Leu Pro Glu Leu Trp Ser
            100                 105                 110

Leu Leu Gly His Ile Asp Ala Val His Gly Leu Tyr Tyr Leu Leu Met
        115                 120                 125

His Gly Trp Phe Ala Ile Phe Pro Pro Thr Glu Leu Trp Ser Arg Leu
    130                 135                 140

Pro Ser Cys Leu Ala Ile Gly Ala Ala Ala Ala Gly Val Val Val Phe

145                 150                 155                 160

Ala Lys Gln Phe Ser Gly Arg Thr Thr Ala Val Cys Ala Gly Ala Val
                165                 170                 175

Phe Ala Ile Leu Pro Arg Val Thr Trp Ala Gly Ile Glu Ala Arg Ser
            180                 185                 190

Ser Ala Leu Ser Val Ala Ala Ala Val Trp Leu Thr Val Leu Leu Val
        195                 200                 205

Ala Ala Val Arg Cys Asn Thr Gln Arg Arg Trp Leu Leu Tyr Ala Leu
    210                 215                 220

Val Leu Met Leu Ser Ile Leu Val Ser Ile Asn Leu Ala Leu Leu Val
225                 230                 235                 240

Pro Ala Tyr Ala Thr Met Val Pro Leu Leu Ala Ser Gly Lys Ser Arg
                245                 250                 255

Lys Ser Pro Val Ile Trp Trp Thr Val Val Thr Ala Ala Ala Leu Gly
            260                 265                 270

Ala Met Thr Pro Phe Ile Leu Phe Ala His Gly Gln Val Trp Gln Val
        275                 280                 285

Gly Trp Ile Ala Gly Leu Asn Arg Asn Ile Ile Leu Asp Val Ile His
    290                 295                 300

Arg Gln Tyr Phe Asp His Ser Val Pro Phe Ala Ile Leu Ala Gly Leu
305                 310                 315                 320

Ile Val Ala Ala Gly Ile Ala Ala His Leu Ala Gly Ala Arg Gly Pro
                325                 330                 335

Gly Gly Asp Thr His Arg Leu Val Leu Val Ser Ala Ala Trp Ile Val
            340                 345                 350

Val Pro Thr Ala Val Val Leu Ile Tyr Ser Ala Thr Val Glu Pro Ile
        355                 360                 365

Tyr Tyr Pro Arg Tyr Leu Ile Leu Thr Ala Pro Ala Ala Ala Val Ile
    370                 375                 380

Leu Ala Val Cys Val Val Thr Ile Ala Arg Lys Pro Trp Leu Ile Ala
385                 390                 395                 400

Gly Val Val Phe Leu Leu Ala Ala Ala Ala Phe Pro Asn Tyr Phe Phe
                405                 410                 415

Thr Gln Arg Gly Pro Tyr Ala Lys Glu Gly Trp Asp Tyr Ser Gln Val
            420                 425                 430

Ala Asp Val Ile Ser Ala His Ala Lys Pro Gly Asp Cys Leu Leu Val
        435                 440                 445

Asp Asn Thr Ala Gly Trp Arg Pro Gly Pro Ile Arg Ala Leu Leu Ala
    450                 455                 460

Thr Arg Pro Ala Ala Phe Arg Ser Leu Ile Asp Val Glu Arg Gly Thr
465                 470                 475                 480

Tyr Gly Pro Lys Val Gly Thr Leu Trp Asp Gly His Val Ala Val Trp
                485                 490                 495

Leu Thr Thr Ala Lys Ile Asp Lys Cys Pro Thr Leu Trp Thr Ile Ala
            500                 505                 510

Asn Arg Asp Lys Ser Leu Pro Asp His Gln Val Gly Glu Met Leu Ser
        515                 520                 525

Pro Gly Thr Gly Phe Gly Arg Thr Pro Val Tyr Arg Phe Pro Ser Tyr
    530                 535                 540

Leu Gly Phe Arg Ile Val Glu Arg Trp Gln Phe His Tyr Ser Gln Val
545                 550                 555                 560

Val Lys Ser Thr Arg
                565


<210>  3
<211>  556
<212>  PRT
<213>  Mycobacterium bovis

<400>  3

Met His Ala Ser Arg Pro Gly Ala Pro Pro His Ala Gly Leu Pro Ser
1               5                   10                  15

Arg Arg Thr Ala Gly Asp Gln Asp His Arg Ala Asp Pro Lys Val Thr
            20                  25                  30

Arg Ile Met Ser Ala Ser Thr Leu Glu Gln Pro Ala Ala Ala His Val
        35                  40                  45

Asp Glu Leu Val Ala Arg Met Arg Gly Arg Leu Leu Asp Pro Leu Ala
    50                  55                  60

Ile Ala Val Leu Ala Ala Val Ile Ser Gly Ala Trp Ala Ser Arg Pro
65                  70                  75                  80

Ser Leu Trp Phe Asp Glu Gly Ala Thr Ile Ser Ala Ser Ala Ser Arg
                85                  90                  95

Thr Leu Pro Glu Leu Trp Ser Leu Leu Gly His Ile Asp Ala Val His
            100                 105                 110

Gly Leu Tyr Tyr Leu Leu Met His Gly Trp Phe Ala Ile Phe Pro Pro
        115                 120                 125

Thr Glu Leu Trp Ser Arg Leu Pro Ser Cys Leu Ala Ile Gly Ala Ala
    130                 135                 140

Ala Ala Gly Val Val Val Phe Ala Lys Gln Phe Ser Gly Arg Thr Thr
145                 150                 155                 160

Ala Val Cys Ala Gly Ala Val Phe Ala Ile Leu Pro Arg Val Thr Trp
                165                 170                 175

Ala Gly Ile Glu Ala Arg Ser Ser Ala Leu Ser Val Ala Ala Ala Val
            180                 185                 190

Trp Leu Thr Val Leu Leu Val Ala Ala Val Arg Cys Asn Thr Gln Arg
        195                 200                 205

Arg Trp Leu Leu Tyr Ala Leu Val Leu Met Leu Ser Ile Leu Val Ser
    210                 215                 220

Ile Asn Leu Ala Leu Leu Val Pro Ala Tyr Ala Thr Met Val Pro Leu
225                 230                 235                 240

Leu Ala Ser Gly Lys Ser Arg Lys Ser Pro Val Ile Trp Trp Thr Val
                245                 250                 255

Val Thr Ala Ala Ala Leu Gly Ala Met Thr Pro Phe Ile Leu Phe Ala
            260                 265                 270

His Gly Gln Val Trp Gln Val Gly Trp Ile Ala Gly Leu Asn Arg Asn
        275                 280                 285

Ile Ile Leu Asp Val Ile His Arg Gln Tyr Phe Asp His Ser Val Pro
    290                 295                 300

Phe Ala Ile Leu Ala Gly Leu Ile Val Ala Ala Gly Ile Ala Ala His
305                 310                 315                 320

Leu Ala Gly Ala Arg Gly Pro Gly Gly Asp Thr His Arg Leu Val Leu
                325                 330                 335

Val Ser Ala Ala Trp Ile Val Val Pro Thr Ala Val Val Leu Ile Tyr
            340                 345                 350

Ser Ala Thr Val Glu Pro Ile Tyr Tyr Pro Arg Tyr Leu Ile Leu Thr
        355                 360                 365

Ala Pro Ala Ala Ala Val Ile Leu Ala Val Cys Val Val Thr Ile Ala

370                 375                 380

Arg Lys Pro Trp Leu Ile Ala Gly Val Val Phe Leu Leu Ala Ala Ala
385                 390                 395                 400

Ala Phe Pro Asn Tyr Phe Phe Thr Gln Arg Gly Pro Tyr Ala Lys Glu
                405                 410                 415

Gly Trp Asp Tyr Ser Gln Val Ala Asp Val Ile Ser Ala His Ala Lys
            420                 425                 430

Pro Gly Asp Cys Leu Leu Val Asp Asn Thr Ala Gly Trp Arg Pro Gly
        435                 440                 445

Pro Ile Arg Ala Leu Leu Ala Thr Arg Pro Ala Ala Phe Arg Ser Leu
    450                 455                 460

Ile Asp Val Glu Arg Gly Thr Tyr Gly Pro Lys Val Gly Thr Leu Trp
465                 470                 475                 480

Asp Gly His Val Ala Val Trp Leu Thr Thr Ala Lys Ile Asp Lys Cys
                485                 490                 495

Pro Thr Leu Trp Thr Ile Ala Asn Arg Asp Lys Ser Leu Pro Asp His
            500                 505                 510

Gln Val Gly Glu Met Leu Ser Pro Gly Thr Gly Phe Gly Arg Thr Pro
        515                 520                 525

Val Tyr Arg Phe Pro Ser Tyr Leu Gly Phe Arg Ile Val Glu Arg Trp
    530                 535                 540

Gln Phe His Tyr Ser Gln Val Val Lys Ser Thr Arg
545                 550                 555

<210>  4
<211>  616
<212>  PRT
<213>  Mycobacterium avium

<400>  4

Met Val Ser Gln Pro Lys Thr Val Ser Arg Leu Leu Asp Ala Glu Glu
1               5                   10                  15

Val Asp Gln Arg His Gly Val Ala Arg Arg Val Cys Gln Phe Gly Gly
            20                  25                  30

Pro Gly Cys Gly Asn Gly Gln Phe Arg Arg Cys Gln Asn Asn Pro Ile
        35                  40                  45

Pro Glu Gly Ser Ala Glu Ser Ile Ala Leu Arg Arg Phe Ser Pro Ile
    50                  55                  60

Ala Gly Pro Thr Ala Asn Ser Pro Ala Ala Val Cys Glu Pro Pro His
65                  70                  75                  80

Arg Ala Arg Pro Lys Val Thr Ser Ile Met Ser Met Pro Thr Leu Glu
                85                  90                  95

Pro Thr Phe Glu Ser Gly Ala Gly Asp Ile Val Ala Glu Pro Ala Pro
            100                 105                 110

Arg Arg Pro Arg Gly Arg Leu Leu Asp Pro Trp Ala Ile Ala Val Leu
        115                 120                 125

Ala Thr Ala Leu Ser Ala Ala Trp Ala Cys Arg Pro Ser Leu Trp Phe
    130                 135                 140

Asp Glu Gly Ala Thr Ile Ser Ala Ala Ala Asn Arg Thr Leu Pro Glu
145                 150                 155                 160

Leu Trp Arg Leu Leu Gly His Ile Asp Ala Val His Gly Ala Tyr Tyr
                165                 170                 175

Leu Leu Met His Gly Trp Phe Ala Leu Phe Pro Pro Thr Glu Phe Phe
            180                 185                 190

Ser Arg Phe Pro Ser Ala Leu Ala Val Gly Ala Ala Ala Ala Gly Val
        195                 200                 205

Thr Val Phe Thr Arg Gln Phe Ala Pro Arg Arg Thr Ala Val Cys Ala
    210                 215                 220

Gly Ala Val Phe Ala Leu Leu Pro Arg Met Thr Trp Ala Gly Met Glu
225                 230                 235                 240

Ala Arg Pro Tyr Ala Phe Val Ala Ala Ala Ala Val Trp Leu Thr Val
                245                 250                 255

Leu Phe Val Ala Ala Val Arg Arg Gly Ala Pro Arg Arg Trp Val Gly
            260                 265                 270

Tyr Ala Leu Ala Leu Met Leu Ala Ile Leu Leu Asn Leu Asn Met Val
        275                 280                 285

Leu Met Val Pro Val Tyr Gly Val Met Leu Pro Leu Leu Thr Ala Arg
    290                 295                 300

Gly Ala Arg Arg Ser Ala Ala Leu Trp Trp Ala Gly Ser Ser Ala Val
305                 310                 315                 320

Ala Val Gly Ala Met Thr Pro Phe Leu Leu Phe Ala His Asn Gln Val
                325                 330                 335

Trp Gln Val Asn Trp Ile Tyr Pro Val Ser Trp His Tyr Ala Phe Asp
            340                 345                 350

Ile Ile Leu Arg Gln Tyr Phe Asp His Ser Val Ala Leu Ala Val Leu
        355                 360                 365

Ser Ala Val Leu Ile Val Ala Ala Ala Val Ala Arg Leu Ala Gly Val
    370                 375                 380

Pro Ala Pro Pro Gly Asp Leu Arg Arg Leu Leu Ile Leu Cys Ala Ala
385                 390                 395                 400

Trp Met Val Ile Pro Thr Ala Leu Val Val Val Tyr Ser Ala Val Gly
                405                 410                 415

Glu Pro Ile Tyr Tyr Pro Arg Tyr Leu Ile Phe Thr Ala Pro Ala Met
            420                 425                 430

Ala Ile Val Leu Ala Val Cys Ile Val Thr Leu Ala Arg Arg Pro Trp
        435                 440                 445

Pro Ile Ala Gly Ala Val Leu Leu Cys Ala Val Ala Ala Leu Pro Asn
    450                 455                 460

Tyr Leu Phe Val Gln Arg Trp Pro Tyr Ala Lys Glu Gly Trp Asp Tyr
465                 470                 475                 480

Ser Gln Val Ala Asp Leu Ile Gly Ser His Ala Ala Pro Gly Asp Cys
                485                 490                 495

Leu Leu Val Asp Asn Thr Val Pro Trp Arg Pro Gly Pro Ile Arg Ala
            500                 505                 510

Leu Leu Ala Thr Arg Pro Ala Ala Phe Arg Ser Leu Ile Asp Val Glu
        515                 520                 525

Arg Gly Ala Tyr Gly Pro Lys Val Gly Thr Leu Trp Asp Gly His Val
    530                 535                 540

Ala Ile Trp Leu Thr Thr Ala Lys Ile Asn Lys Cys Ser Thr Ile Trp
545                 550                 555                 560

Thr Ile Thr Asn Lys Asp Asn Ser Leu Pro Asp His Gln Ser Gly Gln
                565                 570                 575

Ser Leu Pro Pro Gly Ser Ala Phe Gly Gln Ala Pro Ala Tyr Arg Phe
            580                 585                 590

Pro Gly Tyr Leu Gly Phe His Ile Val Glu Arg Trp Gln Phe His Tyr
        595                 600                 605

Ser Gln Val Val Lys Ser Thr Arg

610                 615

<210>  5
<211>  527
<212>  PRT
<213>  Mycobacterium leprae

<400>  5

Met Thr Ser Ile Met Ser Val Ser Ala Leu Glu Gln Ser Ala Ala Asp
1               5                   10                  15

Val Gly Asp Asn Ser Ala Arg Gln His Ala His Gly Ala Leu Pro Asp
            20                  25                  30

Ser Leu Ala Ile Ala Met Leu Ala Thr Val Ile Ser Gly Ala Trp Ala
        35                  40                  45

Ser Arg Pro Ser Leu Trp Phe Asp Glu Ala Ala Thr Ile Ser Ala Ser
    50                  55                  60

Ala Ser Arg Thr Val Pro Glu Leu Trp Arg Leu Leu Ser His Ile Asp
65                  70                  75                  80

Ala Val His Gly Leu Tyr Tyr Leu Leu Met His Gly Trp Phe Ala Ile
                85                  90                  95

Phe Pro Ser Thr Glu Phe Trp Ser Arg Val Pro Ser Cys Leu Ala Ile
            100                 105                 110

Gly Ala Ala Ala Ala Gly Val Thr Val Phe Thr Arg Gln Phe Ala Thr
        115                 120                 125

Arg Thr Thr Ala Val Tyr Ala Gly Ile Val Phe Ala Ile Leu Pro Arg
    130                 135                 140

Ile Thr Trp Ala Gly Ile Glu Ala Arg Ser Ser Ala Leu Ser Val Ala
145                 150                 155                 160

Ala Ala Met Trp Leu Thr Val Leu Leu Val Ala Ser Val Gln Arg Asn
                165                 170                 175

Arg Pro Arg Leu Trp Leu Cys Tyr Ala Leu Thr Leu Met Leu Ser Ile
            180                 185                 190

Leu Leu Asn Leu Thr Leu Ala Thr Leu Val Leu Val Tyr Ala Val Ile
        195                 200                 205

Leu Pro Trp Leu Ala Pro Asn Lys Phe Arg Asn Ser Pro Phe Ile Trp
    210                 215                 220

Trp Ala Val Thr Ser Val Val Ala Leu Gly Thr Ile Thr Pro Phe Ile
225                 230                 235                 240

Leu Phe Ala His Gly Gln Val Trp Gln Val Asp Trp Ile Phe Arg Val
                245                 250                 255

Ser Trp His Tyr Val Phe Asp Ile Thr Gln Arg Gln Tyr Phe Asp His
            260                 265                 270

Ser Val Ser Phe Ala Ile Ala Thr Ala Val Ile Ile Val Pro Ala Ile
        275                 280                 285

Ala Thr Arg Leu Ala Gly Leu Arg Ala Pro Ala Gly Asp Leu Arg Ser
    290                 295                 300

Leu Val Ile Ile Cys Thr Ala Trp Ile Val Ile Pro Thr Thr Leu Met
305                 310                 315                 320

Val Gly Tyr Ser Ala Val Ile Glu Pro Val Tyr Tyr Pro Arg Tyr Leu
                325                 330                 335

Ile Leu Thr Ala Pro Ala Ala Ala Ile Val Ile Ala Val Cys Ile Val
            340                 345                 350

Thr Val Ala Arg Lys Pro Trp Pro Ile Ala Gly Val Leu Val Leu Phe
        355                 360                 365

Ala Val Ala Ala Phe Pro Asn Tyr Leu Phe Thr Gln Arg Gly Arg Tyr
    370                 375                 380

Ala Lys Glu Gly Trp Asp Tyr Ser Gln Val Ala Asp Val Ile Ser Ser
385                 390                 395                 400

Gln Ala Ala Pro Gly Asp Cys Leu Ile Val Asp Asn Thr Val Pro Trp
                405                 410                 415

Arg Pro Gly Pro Ile Arg Ala Leu Leu Ala Ala Arg Pro Ala Ala Phe
            420                 425                 430

Arg Ser Leu Ile Asp Ile Glu Arg Gly Phe Tyr Gly Pro Thr Val Gly
        435                 440                 445

Thr Leu Trp Asp Gly His Val Pro Val Trp Leu Val Thr Ala Lys Ile
    450                 455                 460

Asn Lys Cys Ser Thr Val Trp Thr Val Ser Asp Arg Asp Thr Ser Leu
465                 470                 475                 480

Pro Asp His Gln Ala Gly Gln Leu Leu Ser Pro Gly Leu Ile Leu Gly
                485                 490                 495

Arg Ala Pro Ala Tyr Gln Phe Pro Ser Tyr Leu Gly Phe Arg Ile Val
            500                 505                 510

Glu Arg Trp Gln Phe His Tyr Ser Gln Val Ile Lys Ser Thr Arg
        515                 520                 525

<210>  6
<211>  541
<212>  PRT
<213>  Mycobacterium avium

<400>  6

Met Tyr Pro Ile Asp Ala Ser Gly Ser Ile Asp Arg Ser Tyr Val Arg
1               5                   10                  15

Ile Pro Gly Cys Pro Ser Pro Pro Gly Gly Pro Thr Gly Val Trp Gln
            20                  25                  30

His Gly Gly Val Ser Ser Pro Thr Arg Pro Ala Val Pro Ala Ala Leu
        35                  40                  45

Asp Pro Trp Ile Val Ala Ala Leu Ala Ala Ala Val Ser Leu Ala Gly
    50                  55                  60

Ala Ala Arg Pro Ser Phe Trp Tyr Asp Glu Ala Ala Thr Ile Ser Ala
65                  70                  75                  80

Ser Tyr Ser Arg Ser Leu Thr Gln Met Trp His Met Leu Gly Asn Val
                85                  90                  95

Asp Ala Val His Gly Leu Tyr Tyr Leu Leu Met His Gly Trp Phe Arg
            100                 105                 110

Leu Val Pro Pro Thr Glu Phe Trp Ser Arg Ala Pro Ser Gly Leu Ala
        115                 120                 125

Val Gly Ala Ala Ala Ala Gly Val Val Val Leu Gly Arg Gln Phe Ser
    130                 135                 140

Ser Arg Thr Val Ala Val Val Ser Gly Val Phe Cys Ala Val Leu Pro
145                 150                 155                 160

Arg Thr Thr Trp Ala Gly Val Glu Ala Arg Pro Tyr Ala Leu Ser Met
                165                 170                 175

Met Ala Ala Val Trp Leu Ser Val Leu Leu Val Phe Ala Ala Arg Arg
            180                 185                 190

Gln Ser Arg Trp Pro Trp Leu Cys Phe Gly Leu Ala Leu Val Cys Ser
        195                 200                 205

Val Leu Leu Asp Ala Tyr Ile Ala Leu Leu Leu Ala Ala Tyr Ala Val
    210                 215                 220

Phe Val Gly Val Cys Cys Arg Thr Arg Thr Val Leu Trp Arg Phe Gly
225                 230                 235                 240

Ile Ser Ser Ala Val Ala Val Gly Val Leu Leu Pro Phe Leu Leu Thr
                245                 250                 255

Val Ala Gly Gln Ala His Gln Ile Ser Trp Val Ala Ser Ile Gly His
            260                 265                 270

Arg Thr Val Glu Asp Val Val Met Gln Gln Tyr Phe Glu Arg Ser Pro
        275                 280                 285

Pro Phe Ala Val Leu Ser Ala Leu Leu Ile Cys Ala Ala Ile Ala Leu
    290                 295                 300

Trp Leu Ser Arg Ser Ala Pro Pro Gly Pro Ser Glu Arg Gln Leu Leu
305                 310                 315                 320

Val Leu Ala Thr Cys Trp Val Gly Ile Pro Thr Ala Ala Ile Val Ala
                325                 330                 335

Tyr Ser Ala Leu Val His Pro Ile Tyr Thr Pro Arg Tyr Leu Cys Phe
            340                 345                 350

Thr Ala Pro Ala Met Ala Leu Ile Leu Gly Val Cys Ser Ala Ala Ile
        355                 360                 365

Ala Ala Lys Pro Trp Val Thr Thr Ala Val Val Gly Val Phe Ala Ile
    370                 375                 380

Ala Ala Val Pro Asn Tyr Val Arg Ala Gln Arg Asn Pro Tyr Ala Lys
385                 390                 395                 400

Tyr Gly Met Asp Tyr Ser Gln Val Ala Asp Leu Ile Thr Ala Lys Ala
                405                 410                 415

Ala Pro Gly Asp Cys Leu Leu Val Asn Asp Thr Val Thr Phe Met Pro
            420                 425                 430

Ala Pro Met Arg Pro Leu Leu Ala Ala Arg Pro Asp Ala Tyr Arg Lys
        435                 440                 445

Leu Ile Asp Leu Thr Leu Trp Gln Arg Ala Val Asp Arg Asn Asp Val
    450                 455                 460

Phe Asp Thr Asn Leu Ile Pro Glu Val Val Ala Gly Pro Leu Ser His
465                 470                 475                 480

Cys Ala Val Leu Trp Ile Ile Thr Gln Ala Asp Pro Ser Glu Pro Ala
                485                 490                 495

His Gln Gln Gly Pro Ala Leu Pro Pro Gly Pro Val Tyr Gly Ala Thr
            500                 505                 510

Pro Ala Phe Ala Val Pro His Asp Leu Gly Phe Arg Leu Val Glu Arg
        515                 520                 525

Trp Gln Phe Asn Leu Val Gln Val Phe Glu Ala Thr Lys
    530                 535                 540

<210>  7
<211>  1614
<212>  DNA
<213>  Mycobacterium marinum

<400>  7
atgacgcgtg accaggatca tcgctgggcc cttacagtga caagcatcat gtccacaccg      60

acgctcgagc agggcgcgac ggctaccgac aaggccgagc gcgtacgccc acgtggccgg     120

cggctcgacc cgtggttgat ggctttgctg gcaacgctga tcagcgtcgc ctgggccggt     180

aagccctctc tgtggttcga cgagggcgca acgatttcgg cggccgcgaa ccggacgttg     240

ccggagctgt ggaaactgct gggccacatc gatgccgtgc atggcttcta ctacctgctg     300

atgcacggct ggtatgcgat ctttccgccg acggaattct ggtcgcgggt gcccagcagc     360

ttggccatcg gcggtgccgc cgcgggtgta gtcgtgttca ccaagcagtt ctccggacgc     420

tccaccgcgt tgtgcgcggg tgccgtcttt gccatcctgc cgcgggtgac gtgggcggga     480

atcgaggccc gctcctcggc cttgtcggtg gcggccgcgg tctggctgac cgtgctgctg     540

gtcgccgcgg tgcggcgtaa ccggccatgg ctatggctgc tctacgcgct ggtgttgatg     600

gtgtcgatcc tgatcaacat caacctgggg ttgttggtgc ccgtctatgg cgcgctgcta     660

ccgctgctgg cccccaagaa atctcgaaaa tctcccggca tctggtgggc cgtcacctcg     720

gtggtcgcga ttggggcaat gacgccgttc gtgctgttcg cgcacgggca ggtttggcag     780

gtcggatgga tctcggggct gaaccgcaat ctgttcctgg acgtggtgca tcgccaatac     840

ttcgaccaca gtgtcgcgtt cgccatcctg gccggcgtgc ttgtcgtcgc cgccatcgcc     900

gtgcggctga ccggcatagc gaggccgggc gaggggacgc gcccgctact gctggccagc     960

atcgcttggg ttgtcatacc cactggcatc gttctgctgt actcggcgat cgttgaaccg    1020

atctactacc cccgctatct gatcctcacc gccccggcgg ccgccgtcat cctggcggtg    1080

tgcatcgtca cgcttgcccg caaaccctgg ctcatcaccg gtgtcgtggt gctgctggcc    1140

gtcgcggcgt tcccgaacta tttcttcacc caacgcggcc cgtacgccaa ggagggttgg    1200

gactacagcc aagtggccga tgtcatcagc gcccatgccg caccgggaga ctgtctgctg    1260

gtggacaaca ccgtgccctg gaagccgggg ccggtccgcg cgctactggc cacccggccg    1320

gccgcgttcc ggtcgctgat cgacgtggag cgcggcgcct acgggcccaa ggtcggcacc    1380

ttgtgggacg gccatgttgc cgtgtggctg accacggaga agatcaacaa gtgctcgacg    1440

ttgtggacga tctccaatcg cgacaacgca cttcctgacc atgaagtcgg gccggcgttg    1500

tcgccgggga cggcgttcgg caaaaccccg gtctacctat tcccccacta cctgggattc    1560

cacatcgtcg agcgatggca gttccactac tcgcaggtca tcaagtcaac ccgg          1614

<210>  8
<211>  1671
<212>  DNA
<213>  Mycobacterium bovis

<400>  8
atgcatgcga gtcgtcccgg cgcaccgccg cacgccgggc tgcccagtcg ccgaaccgcc      60

ggtgaccagg atcatcgcgc ggaccctaag gtgacccgca tcatgtccgc ctccactctg     120

gagcagcccg cggcagcaca cgtcgacgag ttggtggcgc ggatgcgcgg ccggctgctc     180

gacccgctgg cgattgcagt gctggccgcg gtcatcagcg gcgcctgggc aagcaggcca     240

tcgttgtggt tcgacgaggg ggcaacgatc tcggcttcag ccagccggac attgccagag     300

ctatggagtc tgctgggcca tatcgacgcc gtgcacggcc tgtactacct gttgatgcat     360

ggctggttcg ccatatttcc gcccaccgaa ttatggtcgc ggcttcccag ctgcctggcc     420

attggagcgg ccgccgccgg cgtggtggtt tttgccaaac agttttcggg acgcaccacg     480

gcggtgtgtg cgggagccgt gttcgcgatt ctgcccaggg tgacgtgggc cggaatcgaa     540

gcacgctcct ccgcgctgtc ggtagcagcc gccgtctggc tgaccgtatt actcgtggcc     600

gcggtgcggt gcaacaccca gcggcggtgg ctgctctacg cgctggtttt gatgctgtcg     660

atcttggtca gtatcaacct ggccctgttg gtaccggcct atgcgacgat ggtgccgctg     720

ctggcgtccg ggaaatcacg caaatctccc gtgatctggt ggacggtcgt cacggcagcc     780

gcgctcgggg ccatgacacc gttcatactg ttcgcccacg gccaggtttg gcaggtcggg     840

tggatcgcag ggttgaacag aaacatcatt ctcgacgtca tacaccgcca gtatttcgat     900

cacagtgttc cgttcgccat cctcgcgggc ctcatcgtcg ctgccggcat cgcggcgcat     960

ctggccggag ctcgtggacc cggtggcgat acccaccggc tcgtgctcgt cagcgcagcc    1020

tggatcgtcg tgcccaccgc cgtcgtcctc atctactcgg cgaccgtcga accgatctac    1080

tacccgcgct acctgatcct caccgccccc gccgcggccg tcatcctggc ggtttgcgtc    1140

gtcaccatcg cccgcaagcc gtggctcatc gccggggtcg tgtttctcct tgccgccgca    1200

gcgtttccga actacttctt cacacagcgg gggccgtacg cgaaagaggg ctgggattac    1260

agccaggtgg cagatgtcat cagcgcccat gccaagcccg gggattgcct gctggtggac    1320

aacaccgcgg gttggcgacc cgggcccatc cgcgccctgc tggccacccg gccggcggcg    1380

ttccggtcgc tgattgacgt cgagcgcggc acctacggcc ccaaggtcgg cactttgtgg    1440

gatggccatg tcgctgtgtg gcttacgacg gccaagatcg acaagtgccc cacgctgtgg    1500

acgatagcca atcgtgacaa gtcgttgccc gatcatcagg tcggcgaaat gttgtcaccg    1560

ggaacaggct tcgggcgcac gcccgtatac cggttcccga gctacctcgg cttccgcatc    1620

gtcgagcgct ggcagttcca ctactcgcag gtggtcaagt caacgcggta a             1671

## Description

### Field of the invention

The present invention relates to mycobacterial lipoarabinomannan cap-specific mannosyl transferases and nucleic acid encoding such transferases. The invention further relates to Mycobacteria that are deficient in lipoarabinomannan cap-specific mannosyl transferases and that express mannose cap-deficient lipoarabinomannans.

### Background of the invention

Tuberculosis (TB) kills approximately 2 million persons each year. The disease is caused by the bacterium *Mycobacterium tuberculosis.* The vaccine that currently is in use is, however, not considered to be adequate. This vaccine is referred to as BCG (Bacille Calmette-Guérin, after the two French scientists who developed it) and it consists of an attenuated strain of *M. bovis* (a close relative of *M. tuberculosis*). The BCG vaccine was developed more than 80 years ago, and protects mainly against childhood TB, and not against pulmonary disease later in life. Moreover, protective efficacy varies widely and in some studies efficacy in fact was found to be zero.

Recent data suggest that the ability of BCG to cause immunosuppression is a major factor hindering adequate protective immunity. This ability to immunosuppress is also found in TB itself: while more than one third of the world's population is infected, "only" 10% actually develops active TB, while in the other 90% the bacterium resides in an inactive (dormant) state inside macrophages in the form of so-called granuloma's, i.e. fused macrophages with intracellular mycobacteria, surrounded by immune cells (B- and T-cells) and an outermost layer of fibroblast thus "encapsulating" the infected foci. In this form *M. tuberculosis* persists in a lifelong truce with the host.

Mycobacteria express a surface glycolipid called lipoarabinomannan (LAM; 1) and there is ample *in vitro* evidence that LAM contributes to bacterial persistence by alternatively activating macrophages and/or dendritic cells (DC) such that the host immune response is crippled, which results in immunosuppression (4,11,15). LAM of certain mycobacterial species, including *M. tuberculosis,* is biosynthesized with a so-called mannose cap. This mannose cap is a short mannan chain (of one, two or three mannoses) on the non-reducing end of the arabinan domain. Mannose-capped LAM is also referred to as manLAM. The non-pathogenic mycobacterium *M. smegmatis* expresses a LAM that naturally lacks a mannose cap (also referred to as araLAM).

Apart form the diverse effects that manLAM has versus araLAM on cytokine profiles of DC, two research groups have found that manLAM but not araLAM inhibits fusion of phagosomes with lysosomes (12, 13). Inhibition of phago-lysososome fusion is seen as one of the hallmarks of infection with pathogenic mycobacteria. Thus, two lines of evidence suggest an important role of LAM in mycobacterial pathogenesis. However, until now all studies have been done with purified manLAM (from *M. tuberculosis* or *M. bovis*) and araLAM (from *M. smegmatis*), and these LAMs cannot be considered to be isogenic. In particular araLAM from *M. smegmatis* and manLAM from *M. tuberculosis* or *M. bovis* structurally differ in more than only the absence or presence of a mannose cap. Therefore it cannot be excluded that the observed differences in biological effects between of manLAM and araLAM are caused by other differences in LAM structure than the mannose cap.

Apart from playing a role in bacterial persistence, the presence of a mannose cap may be of relevance to the inability of current TB vaccines tot elicit an effective immune response. There is ample evidence that the ability of the BCG vaccine to cause immunosuppression is a major factor hindering adequate protective immunity (6, 3, 9, 7). There is thus a need to investigate whether a capless BCG mutant would cause less immunosuppression and hence would serve as a more effective vaccine against TB.

However, at present it is not known which mycobacterial genes and gene products (enzymes) are involved in the biosynthesis of the manLAM mannose cap. Let alone that it is known that viable capless mutants can be obtained.

It is thus an object of the present invention to provide for nucleotide and amino acid sequences involved in the biosynthesis of mycobacterial manLAM mannose caps. It is a further object of the invention to provide for novel mycobacterial mutants that are deficient in the biosynthesis of manLAM mannose caps and to provide for effective vaccines that comprises such mutants or components thereof as well as methods for their production.

### Description of the invention

### Definitions:

The term "gene" means a DNA fragment comprising a region (transcribed region), which is transcribed into an RNA molecule (e.g. an mRNA) in a cell, operably linked to suitable regulatory regions (e.g. a promoter). A gene may thus comprise several operably linked fragments, such as a promoter, a 5' leader sequence, a coding region and a 3'nontranslated sequence (3'end) comprising e.g. transcription termination sequences. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide or which is active itself (e.g. in post-transcriptional gene silencing or RNAi). The 5'-end of the coding sequence may encode a (homologous or heterologous) secretion signal, so that the encoded protein or peptide is secreted out of the cell. The coding sequence is preferably in sense-orientation and encodes a desired, biologically active protein or protein fragment.

A "chimeric" or "recombinant" gene refers to any gene, which is not normally found in nature in a species, in particular a gene in which different parts of the nucleic acid region are not associated in nature with each other. For example the promoter is not associated in nature with part or all of the transcribed region or with another regulatory region. The term "chimeric gene" is understood to include expression constructs in which a promoter or transcription regulatory sequence is operably linked to one or more coding sequences.

The term "nucleic acid sequence" (or nucleic acid molecule) refers to a DNA or RNA molecule in single or double stranded form, particularly a DNA encoding a protein or protein fragment according to the invention. An "isolated nucleic acid sequence" refers to a nucleic acid sequence which is no longer in the natural environment from which it was isolated, e.g. the nucleic acid sequence in a bacterial host cell or in the plant nuclear or plastid genome.

A "nucleic acid construct" or "nucleic acid vector" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules.

The term peptide herein refers to any molecule comprising a chain of amino acids that are linked in peptide bonds. The term peptide thus includes oligopeptides, polypeptides and proteins, including multimeric proteins, without reference to a specific mode of action, size, 3-dimensional structure or origin. A "fragment" or "portion" of a protein may thus still be referred to as a "protein". An "isolated protein" is used to refer to a protein which is no longer in its natural environment, for example *in vitro* or in a recombinant host cell. The term peptide also includes post-expression modifications of peptides, e.g. glycosylations, acetylations, phosphorylations, and the like. A "truncated protein" refers herein to a protein which is reduced in amino acid length compared to the wild type protein.

A "chimeric protein" or "hybrid protein" is a protein composed of various protein "domains" (or motifs) which is not found as such in nature but which a joined to form a functional protein, which displays the functionality of the joined domains (for example receptor binding). A chimeric protein may also be a fusion protein of two or more proteins occurring in nature. The term "domain" as used herein means any part(s) or domain(s) of the protein with a specific structure or function that can be transferred to another protein for providing a new hybrid protein with at least the functional characteristic of the domain.

The term "expression vector" refers to nucleotide sequences that are capable of affecting expression of a gene in host cells or host organisms compatible with such sequences. These expression vectors typically include at least suitable transcription and translation regulatory sequences and optionally, 3' transcription termination signals. DNA encoding the polypeptides of the present invention will typically be incorporated into the expression vector. The expression vector will be introduced into a suitable host cell and be able to effect expression of the coding sequence in an *in vitro* cell culture of the host cell. The expression vector preferably is suitable for replication in a prokaryotic host.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically regulated, e.g. by the application of a chemical inducer.

The term "selectable marker" is a term familiar to one of ordinary skill in the art and is used herein to describe any genetic entity which, when expressed, can be used to select for a cell or cells containing the selectable marker. Selectable markers may be dominant or recessive or bidirectional. The selectable marker may be a gene coding for a product which confers antibiotic resistance to a cell expressing the gene or a non-antibiotic marker gene, such as a gene relieving other types of growth inhibition, i.e. a marker gene which allow cells containing the gene to grow under otherwise growth-inhibitory conditions. Examples of such genes include a gene which confers prototrophy to an auxotrophic strain, e.g. dal genes introduced in a dal⁻ strain (cf. B. Diderichsen in Bacillus: Molecular Genetics and Biotechnology Applications, A. T. Ganesan and J. A. Hoch, Eds., Academic Press, 1986, pp. 35-46) or a thy gene introduced in a thy⁻-cell (cf. Gryczan and Dubnau (1982), Gene, 20, 459-469) or a gene which enables a cell harbouring the gene to grow under specific conditions such as an amdS gene, the expression of which enables a cell harbouring the gene to grow on acetamide as the only nitrogen or carbon source (e.g. as described in EP 635 574), or a gene which confers resistance towards a heavy metal (e.g. arsenite, arsenate, antimony, cadmium or organo-mercurial compounds) to a cell expressing the gene. Cells surviving under these conditions will either be cells containing the introduced DNA construct in an extrachromosomal state or cells in which the above structure has been integrated. Alternatively, the selectable marker gene may be one conferring immunity to a cell expressing the gene. The term "reporter" may be used interchangeably with marker, although it is mainly used to refer to visible markers, such as green fluorescent protein (GFP).

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

The term "ortholog" of a gene or protein refers herein to the homologous gene or protein found in another species, which has the same function as the gene or protein, but is (usually) diverged in sequence from the time point on when the species harbouring the genes diverged (i.e. the genes evolved from a common ancestor by speciation).

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence and, if applicable, another (heterologous) secretory signal sequence and/or terminator sequence than in its natural environment. It is understood that the regulatory sequences, signal sequences, terminator sequences, etc. may also be homologous to the host cell. In this context, the use of only "homologous" sequence elements allows the construction of "self-cloned" genetically modified organisms (GMO's).

"Self-cloning" is defined herein as in European Directive 98/81/EC Annex II: Self-cloning consists in the removal of nucleic acid sequences from a cell of an organism which may or may not be followed by reinsertion of all or part of that nucleic acid (or a synthetic equivalent) with or without prior enzymic or mechanical steps, into cells of the same species or into cells of phylogenetically closely related species which can exchange genetic material by natural physiological processes where the resulting micro-organism is unlikely to cause disease to humans, animals or plants. Self-cloning may include the use of recombinant vectors with an extended history of safe use in the particular micro-organisms.

When used to indicate the relatedness of two nucleic acid sequences the term "homologous" means that one single-stranded nucleic acid sequence may hybridise to a complementary single-stranded nucleic acid sequence. The degree of hybridisation may depend on a number of factors including the amount of identity between the sequences and the hybridisation conditions such as temperature and salt concentration as discussed later.

The term "substantially identical", "substantial identity" or "essentially similar" or "essential similarity" means that two peptide or two nucleotide sequences, when optimally aligned, such as by the programs GAP or BESTFIT using default parameters, share at least a certain percentage of sequence identity as defined elsewhere herein. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). It is clear than when RNA sequences are said to be essentially similar or have a certain degree of sequence identity with DNA sequences, thymine (T) in the DNA sequence is considered equal to uracil (U) in the RNA sequence.

Sequence alignments and scores for percentage sequence identity may be determined using computer programs, such as the GCG Wisconsin Package, Version 10.3, available from Accelrys Inc., 9685 Scranton Road, San Diego, CA 92121-3752 USA or the open-source software Emboss for Windows (current version 2.7.1-07). Alternatively percent similarity or identity may be determined by searching against databases such as FASTA, BLAST, etc.

Optionally, in determining the degree of "amino acid similarity", the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

Nucleotide sequences encoding mannosyl transferases of the invention may also be defined by their capability to "hybridise" with the nucleotide sequences of SEQ ID NO. 7 or SEQ ID NO. 8, under moderate, or preferably under stringent hybridisation conditions. "Stringent hybridisation" conditions are herein defined as conditions that allow a nucleic acid sequence of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0.1 M salt, or less, preferably 0.2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity.

"Moderate conditions" are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

"Adjuvants" are herein defined to include any substance or compound that, when used in combination with an antigen, to immunise a mammal, preferably a human, stimulates the immune system, thereby provoking, enhancing or facilitating the immune response against the antigen, preferably without generating a specific immune response to the adjuvant itself Preferred adjuvants enhance the immune response against a given antigen by at least a factor of 1.5, 2, 2.5, 5, 10 or 20, as compared to the immune response generated against the antigen under the same conditions but in the absence of the adjuvant. Tests for determining the statistical average enhancement of the immune response against a given antigen as produced by an adjuvant in a group of animals or humans over a corresponding control group are available in the art. The adjuvant preferably is capable of enhancing the immune response against at least two different antigens. The adjuvant of the invention will usually be a compound that is foreign to a mammal, thereby excluding immunostimulatory compounds that are endogenous to mammals, such as e.g. interleukins, interferons and other hormones.

Lipoarabinomannan (LAM) as well as its related precursors, lipomannan (LM) and phosphatidyl-*myo*-inositol mannosides (PIMs), are glycolipids that are found interspersed in the mycobacterial cell wall. PIMs, LM and LAM are major lipoglycans that are non-covalently attached to the plasma membrane through their phosphatidyl-*myo*-inositol anchor and extend to the exterior of the cell wall. In LAM (and in LM), an α1,6-linked Man*p* backbone substituted at C-2 by single Man*p* units constitutes the mannan domain. The arabinan polymer is a linear α(1→5)-linked arabinofuranosyl backbone punctuated with branched hexa-arabinofuranosides: β-D-Ara*f*-(1→2)-α-D-Ara*f*-(1-]₂→3 3 and → 5 )-α-D-Ara*f*-(1→5)-α-D-Ara*f*- and linear tetra-arabinofuranosides: β-D-Ara*f*-(1→2)-α-D-Ara*f*-(1→5)-α-D-Ara*f*-(1→5)-α-D-Ara*f*→. The mannose caps, which terminate the arabinan domain, consist of a single Man*p* residue, a dimannoside (α-D-Man*p*(1→2)-α-D-Man*p*→) or a trimannoside (α-D-Man*p-*(1→2)-α-D-Man*p*-(1→2)-α-D-Man*p*→). Mannose-capped LAM (manLAM) thus contains mono-, di-, or trimeric mannose residues at the non-reducing end of the arabinan domains of the LAM, whereas in contrast, araLAM lacks these terminal mannose caps.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Detailed description of the invention

In a first aspect the present invention relates to polypeptides that comprise an amino acid sequence of a lipoarabinomannan (LAM) cap-specific mannosyl transferase. The amino acid sequences are preferably mycobacterial amino acid sequences. Preferably the polypeptides of the invention comprise an amino acid sequence that has at least 35, 40, 42, 43, 45, 50, 55, 60, 65, 70, 71, 72, 75, 80, 90, or 95% amino acid identity with at least one of SEQ ID NO. 1 - 6, which comprise the amino acid sequences of lipoarabinomannan cap-specific mannosyl transferase from various *Mycobacteria.*

A preferred polypeptide according to the invention has mycobacterial manLAM cap-specific mannosyl transferase activity. The mycobacterial manLAM cap-specific mannosyl transferase activity is a novel mannosyl transferase having arabinose as acceptor of the transferred mannose residue. The mycobacterial manLAM cap-specific mannosyl transferase activity of the polypeptide may be assayed in a complementation assay by expression of a nucleotide sequence encoding the polypeptide in a *Mycobacterium* that does not express manLAM but araLAM. Examples of suitable *Mycobacteria* that do not express manLAM include e.g. the *M. marinum* capless 2 mutant described herein or strains of *M. smegmatis.* A polypeptide that has mycobacterial manLAM cap-specific mannosyl transferase activity is a polypeptide that has the ability to restore (or induce) biosynthesis of the manLAM mannose cap upon expression of a nucleotide sequence encoding the polypeptide in a *Mycobacterium* that does not express manLAM, whereby such nucleotide sequences are preferably as herein defined below. Successful complementation by the polypeptide (or encoding nucleotide sequence), i.e. restoration of the manLAM mannose cap, may be determined using mannose cap-specific monoclonal antibodies as may be obtained as described in Example 1 herein. Suitable examples of such monoclonal antibodies are e.g. the 56.49.1A and 55.92.1A1 monoclonal antibodies as well as those described by Chatterjee et al. (1992, J Biol Chem. 267: 6234-9). Alternatively, restoration of the manLAM mannose cap may be determined by various methods that can detect the presence or absence of mannose caps on LAM such as e.g. mass-spectrometry and/or chromatography (Chatterjee et al., 1993 Glycobiology 3: 497-506; Prinzis et al., 1993, J Gen Microbiol. 139:2649-58; Khoo et al., 2001, J Biol Chem. 276:3863-71), NMR (Lee et al., 2005, Glycobiology 15:139-51), and capillary electrophoresis (16). Alternatively, the mycobacterial manLAM cap-specific mannosyl transferase activity of the polypeptide may be assayed by using synthetic acceptors that may be synthesised as described by Gadikota et al. (2). Such synthetic mannose-acceptor may e.g. be the non-reducing ends of the arabinan domain in 8-Aminooctyl β-D-arabinofuranoside (referred to as "ara") or 8-Aminooctyl 5-*O*-{3,5-di-*O*-(2-*O*-[β-D-arabinofuranosyl]-α-D-arabinofuranosyl)-α-D-arabinofuranosyl}-α-D-arabinofuranoside (referred to as (ara)₆). The presence of a mannose-cap on these synthetic acceptors may be detected as described above.

A preferred polypeptide of the invention comprising an immunogenic fragment from an amino acid sequence as defined above. The immunogenic fragment preferably comprises at least 4, 6, 9, 12, 15 or 20 contiguous amino acids from the amino acid sequence.

A polypeptide according to the invention preferably is a polypeptide comprising an amino acid sequence from a *Mycobacterium* selected from *M. bovis, M. tuberculosis, M. avium, M. paratuberculosis, M. leprae, M. ulcerans* and *M. marimum* and/or other mycobacterial species that expresses mycobacterial manLAM cap-specific mannosyl transferase activity as defined above. It is understood that an amino acid sequence that is from a particular *Mycobacterium* is an amino acid sequence as it naturally occurs in the *Mycobacterium.* However, the invention does not exclude amino acid sequences that do not occur in nature, e.g. those that comprise substitutions, deletions and/or insertion of one or more amino acids compared to a naturally occurring amino acid sequence. Preferably the polypeptide of the invention is an isolated polypeptide, i.e. isolated from the environment in which it naturally occurs.

In another aspect, the invention relates to a nucleic acid molecule comprising a nucleotide sequence selected from: (a) nucleotide sequence encoding a polypeptide as defined in above; (b) a nucleotide sequence that has at least 30, 35, 40, 45, 50, 55, 60, 65, 70, 75, 80, 90, or 95% nucleotide identity with SEQ ID NO. 7 or 8; (c) a nucleotide sequence the complementary strand of which hybridises to a nucleotide sequence of (a) or (b); and, (d) a nucleotide sequence the sequence of which differs from the sequence of a nucleotide sequence of (c) due to the degeneracy of the genetic code. A preferred nucleotide sequence encodes a polypeptide having mycobacterial manLAM cap-specific mannosyl transferase activity as defined above. Preferably the nucleotide sequence is from a *Mycobacterium* selected from *M. bovis, M. tuberculosis, M. avium, M. paratuberculosis, M. leprae, M. ulcerans* and *M. marimum* and/or other mycobacterial species that expresses mycobacterial manLAM cap-specific mannosyl transferase activity as defined above. It is understood that a nucleotide sequence that is from a particular *Mycobacterium* is a nucleotide sequence as it naturally occurs in the *Mycobacterium.* However, the invention does not exclude nucleotide sequences that do not occur in nature, e.g. those that comprise substitutions, deletions and/or insertion of one or more nucleotides compared to a naturally occurring nucleotide sequence.

A preferred nucleic acid molecule of the invention comprises a fragment of at least 10 contiguous nucleotides from a nucleotide sequence as defined above. Such (oligo)nucleotides may be used as primers for amplification reactions and/or hybridisation probes. Such probes and primers may e.g. be useful in diagnostics methods and/or e.g. in a method for determining whether a nucleotide sequence as defined above (naturally) occurs in a *Mycobacterium.* Preferably the nucleic acid molecule of the invention is an isolated nucleic acid molecule, i.e. isolated from the environment in which it naturally occurs.

The nucleic acid molecule of the invention may be a vector. A preferred vector is a mycobacterial vector such as e.g. a vector as used in the Examples herein (pSMT3; Golanska et al., 1998, Acta Microbiol Pol. 47: 335-43) or (shuttle) vectors as described in WO 91/13157, WO 90/10701, WO 90/00594, US 6,472,213, Jacobs et al. (1987, Nature, 327: 532-535), Snapper et al. (1988, Proc. Natl. Acad. Sci USA, 85: 6987-6991) and Ranes et al. (J. Bacteriol. 1990, 172: 2793-2797). Preferably in the vector the nucleotide sequence encoding a polypeptide as defined above is operably linked to a promoter. Preferably the promoter is capable of driving transcription of the nucleotide sequence in a suitable host cell. In one embodiment, the invention relates to a host cell comprising a vector as just defined herein.

In a further aspect the present invention relates to a mycobacterial cell that is deficient in manLAM cap-specific mannosyl transferase activity, whereas the cell is of a *Mycobacterium* species that naturally expresses manLAM. Preferably, the cell entirely lacks manLAM cap-specific mannosyl transferase activity, although cells that still contain residual transferase activity (e.g. < 10, 5, 2 or 1%) are not excluded from the invention. Preferably the deficiency in manLAM cap-specific mannosyl transferase activity causes the cell to express reduced levels of manLAM, more preferably the level of manLAM is no more than 10, 5, 2 or 1% of the total LAM expressed in the cell, more preferably the level of manLAM is below the detection limit of any of the methods for determining the presence of manLAM as described above.

In a preferred mycobacterial cell according to the invention, the deficiency in manLAM cap-specific mannosyl transferase activity is caused by the inactivation of a cellular gene encoding a polypeptide comprising an amino acid sequence having manLAM cap-specific mannosyl transferase activity as defined above. The cellular gene preferably is a gene as in naturally occurs in the cell. Usually the cellular gene will be a chromosomal gene although plasmid borne genes or other episomal genes are not excluded from the invention as they may occur. The gene may be inactivated by a variety of methods known to the skilled person (see below). Preferably the cellular gene is inactivated by deletion of at least a part of the sequence coding for the manLAM cap-specific mannosyl transferase activity and/or deletion of at least a part of the upstream regulatory sequences of a nucleotide sequence coding for the manLAM cap-specific mannosyl transferase activity as defined above.

A preferred mycobacterial cell of the invention is a cell of a pathogenic *Mycobacterium.* Preferably the mycobacterial cell is a cell of a slow growing *Mycobacterium,* more preferably a slow growing virulent *Mycobacterium.* Slow growing mycobacteria comprising the virulent mycobacteria are generally found to express manLAM as opposed to fast growing atypical mycobacteria that express araLAM. Preferably the mycobacterial cell of the invention is of a mycobacterial species selected from the group consisting of *M. bovis, M. tuberculosis, M. avium, M. paratuberculosis, M. leprae, M. ulcerans* and *M. marimum* and/or other mycobacterial species that expresses mycobacterial manLAM cap-specific mannosyl transferase activity as defined above. The preferred mycobacterial species include any mycobacterial pathogens of man and animals. A further preferred mycobacterial cell of the invention is a cell of an attenuated *Mycobacterium* or attenuated mycobacterial strain, whereby an attenuated mycobacterial strains is understood to mean a strain having a reduced ability to invade and infect cells. One method for producing attenuated mycobacteria is e.g. described in US 6,548,070. This attenuation permits the novel strains provided herein to be used in immunogenic compositions for administration to a host to generate an immune response. A preferred example of an attenuated *Mycobacterium* for use in the present invention is the vaccine strain *M. bovis* bacillus Calmette-Guérin.

In yet another aspect the invention pertains to a method for producing a mycobacterial cell of the invention as defined above. Preferably the method comprising the steps of: (a) transforming a *Mycobacterium* with a nucleic acid construct that comprises: (i) a part of a nucleotide sequence that has at least 30, 35, 40, 45, 50, 55, 60, 65, 70, 75, 80, 90, or 95% nucleotide identity with SEQ ID NO. 7 or 8 or, (ii) a nucleotide sequence that is present in the genome of the *Mycobacterium* within 2 kb of the nucleotide sequence that has at least 30, 35, 40, 45, 50, 55, 60, 65, 70, 75, 80, 90, or 95% nucleotide identity with SEQ ID NO. 7 or 8 and, (b) selecting a transformant that is deficient in manLAM cap-specific mannosyl transferase activity. The nucleic acid construct is a construct for inactivation of the cellular gene encoding a polypeptide comprising an amino acid sequence having manLAM cap-specific mannosyl transferase activity as defined above. The nucleic acid construct may e.g. be a construct for inactivation of the transferase gene through an insertional mutation of the gene (see e.g. US 6,752,994). The insertional mutation of the transferase gene may be effected through illegitimate recombination of DNA into the mycobacterial chromosome, or by homologous recombination, or by the insertion of a mycobacterial transposon into a mycobacterial gene, or by the transfection of a mycobacterium with a vector which includes a pair of inverted repeat sequences and DNA encoding a transposase. More preferably, however, the nucleic acid construct is a construct that inactivates the transferase gene by allelic exchange as described e.g. in US 5,972,700, US 6,096,549 and US 6,271,034. Even more preferably the nucleic acid construct is a construct as described in US 6,423,545 that inactivates the transferase gene by unmarked allelic exchange, creating a deletion in the mycobacterial genome with leaving (a trace of) a selectable marker gene. Another method for generating an unmarked mycobacterial knock-out mutant is described by Parish and Stoker (2000, Microbiology. 146: 1969-75). Transformation of the nucleic acid constructs may be accomplished by any means known to those skilled in the art, such as e.g. electroporation, or by the generation of protoplasts into which the transforming DNA is inserted, followed by regeneration of the cell wall, as described in Jacobs (1987, *supra*) and Snapper (1988, *supra*). Selection of transformants that are deficient in manLAM cap-specific mannosyl transferase activity may be performed in the same manner as described in Example 2 herein.

In a further aspect the invention relates to a method for producing a mycobacterial manLAM that lacks a mannose cap. The manLAM that lacks a mannose cap is in fact an araLAM. However, the method is particularly aimed at producing an araLAM (or manLAM that lacks a mannose cap) that except for the absence of the mannose cap(s) is identical to a manLAM. Preferably the araLAM produced in the method is identical to a manLAM of a slow growing and/or pathogenic *Mycobacterium* as defined above. The method comprising culturing a mycobacterial cell as defined in above or as obtained in a method as defined above, recovery and optionally purification of the mycobacterial manLAM that lacks a mannose cap. The invention thus also relates to a mycobacterial manLAM that lacks a mannose cap and that is obtainable in a method as just described herein. In one particular embodiment of the invention, the mycobacterial manLAM that lacks a mannose cap of the invention is used as an adjuvant or is used for the manufacture of a medicament in addition to an antigen for immunisation against the antigen.

A preferred embodiment of the invention relates to a pharmaceutical composition comprising at least one of: (a) a mycobacterial cell as defined herein above; (b) a mycobacterial cell as obtainable in a method as defined herein above; and, (c) a mycobacterial manLAM that lacks a mannose cap and that is obtainable in a method as defined herein above; and a pharmaceutically acceptable carrier. The pharmaceutical carrier can be any compatible, non-toxic substance suitable to deliver the active ingredients, i.e. the mycobacterial cells or LAM of the invention, to a patient. Pharmaceutically acceptable carriers for intranasal delivery are exemplified by water, buffered saline solutions, glycerin, polysorbate 20, cremophor EL, and an aqueous mixture of caprylic/capric glyceride, and may be buffered to provide a neutral pH environment. Pharmaceutically acceptable carriers for parenteral delivery are exemplified by sterile buffered 0.9% NaCl or 5% glucose optionally supplemented with a 20% albumin. Preparations for parental administration must be sterile. The parental route for administration of the active ingredients is in accord with known methods, e.g. injection or infusion by subcutaneous, intravenous, intraperitoneal, intramuscular, intraarterial or intralesional routes. The compositions of the invention are preferably administered by bolus injection. For oral administration, the active ingredient can be administered in liquid dosage forms, such as elixirs, syrups, and suspensions. Liquid dosage forms for oral administration can contain coloring and flavoring to increase patient acceptance. Methods for preparing parenterally, orally or intranasally administrable compositions are well known in the art and described in more detail in various sources, including, for example, Remington's Pharmaceutical Science (18th ed., Mack Publishing, Easton, PA, 1990) (incorporated by reference in its entirety for all purposes).

A preferred pharmaceutical composition further comprises an adjuvant. A number of adjuvants are well known to one skilled in the art. Suitable adjuvants include incomplete Freund's adjuvant, alum, aluminium phosphate, aluminium hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxy-phosphoryloxy)-ethylamine (CGP 19835A, referred to as MTP-PE), DDA (2 dimethyldioctadecylammonium bromide), polyIC, Poly-A-poly-U, RIBI™, GERBU™, Pam3™, Carbopol™, Specol™, Titermax™, tetanus toxoid, diphtheria toxoid, meningococcal outer membrane proteins, diphtheria protein CRM₁₉₇. Preferred adjuvants comprises a ligand that is recognised by a Toll-like-receptor (TLR) present on antigen presenting cells. Various ligands recognised by TLR's are known in the art and include e.g. lipopeptides (see e.g. WO 04/110486), lipopolysaccharides, peptidoglycans, liopteichoic acids, lipoarabinomannans of the invention, lipoproteins (from mycoplasma or spirochetes), double-stranded RNA (poly I:C), unmethylated DNA, flagellin, CpG-containing DNA, and imidazoquinolines. In addition, if desired, the pharmaceutical composition may contain auxiliary substances such as e.g. wetting or emulsifying agents, pH buffering agents, which enhance the effectiveness of the compositions as immunogens, adjuvants and/or vaccines.

In a further aspect, the invention relates to a method for immunising (vaccinating) a mammal against a *Mycobacterium,* the method comprising administration of a pharmaceutical composition as defined above in an amount effective to raise an immune response against the *Mycobacterium.* The *Mycobacterium* preferably is a pathogenic *Mycobacterium* as described above. The pharmaceutical compositions of the invention are administered in a manner compatible with the dosage formulation, and in such amount as will be therapeutically effective and immunogenic. The quantity to be administered depends on the subject to be treated including, e.g., the capacity of the individual's immune system to induce an immune response. Suitable dosage ranges are of the order of 10⁴ to 10⁶ cfu (colony forming units) at mycobacterial concentration of about 10⁶ cfu/mg. Most preferably, the effective dose is about 10⁵ cfu. The dosage of the composition will depend on the route of administration and will vary according to the age of the patient to be immunised and, to a lesser degree, the size of the person to be immunised. Most preferably, the composition according to the invention is administered via an intradermal route and in a single boost. In the case of patients affected with immunological disorders such as, for example, immunosupressed/deficient patients, each injected dose preferably contains half the weight quantity of the mycobacteria contained in a dose for a healthy patient. In the case of neonates, the dose will be approximately four times less than for an adult, and in the case of young children (4-6 years old), the dose will be approximately half the dose used for an adult healthy patient. In some instances, it will be necessary to proceed with multiple administrations of the composition of the invention, usually not exceeding six administrations, more usually not exceeding four administrations, and preferably one or more, usually at least about three administrations. The administrations will normally be at from two to twelve week intervals, more usually from three to five week intervals. Periodic boosters at intervals of 1-5 years, usually three years, will be desirable to maintain the desired levels of protective immunity.

In a further aspect the invention relates to a mycobacterial cell as defined above and/or mycobacterial manLAM that lacks a mannose cap as defined above, for use as a medicament, e.g. in a method of immunisation as described above. Alternatively, the mycobacterial cell as defined above and/or mycobacterial manLAM that lacks a mannose cap as defined above may be used for the manufacture of a medicament for the treatment or prophylaxis of a mycobacterial infection.

In yet a further aspect the invention relates to the use of a polypeptide according to the invention that has mycobacterial manLAM cap-specific mannosyl transferase activity, a nucleotide sequence according to the invention that encodes the mycobacterial manLAM cap-specific mannosyl transferase, and/or a host cell that expresses the polypeptide (or the nucleotide sequence) in a method for screening or identification of a compound that inhibits (or at least reduces the activity of) a mycobacterial manLAM cap-specific mannosyl transferase. The method usually comprises contacting the mannosyl transferase and/or a host cell expressing the same and determining the activity of the transferase or determining the binding of the compound to the transferase. A reduced activity as compared to a control sample that does not comprise the compound indicates that the compound inhibits (or at least reduces the activity of) a mycobacterial manLAM cap-specific mannosyl transferase. Preferably a plurality of different compounds is tested, preferably in a high through-put system.

### Description of the Figure

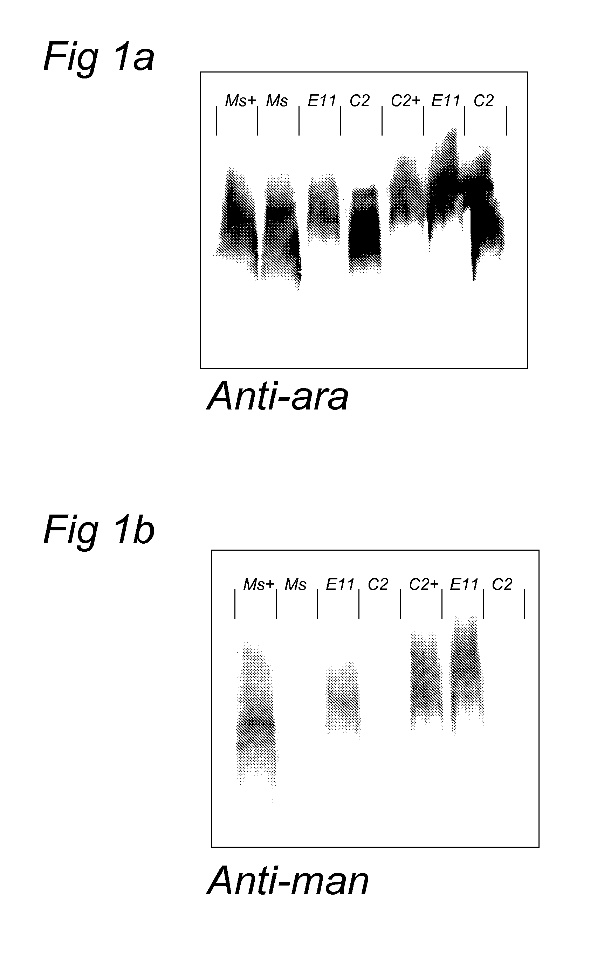

Figure 1 Disrupted cells of *M. barium* E11, capless 2, *M. smegmatis* and complementants of capless 2 and *M. smegmatis* were immunostained with anti-ara (panel A) and anti-cap monoclonal antibodies (panel B), respectively. Ms+ = complementant of *M. smegmatis*; Ms=*M. smegmatis*; E11=*M*. *marinum* parent strain; C2 = capless 2; C2+ = complementant of capless 2. Clear is that capless 2 has lost its cap and that complementation restores cap synthesis. Also is shown that complementation of *M. smegmatis* induces expression of a capped manLAM.

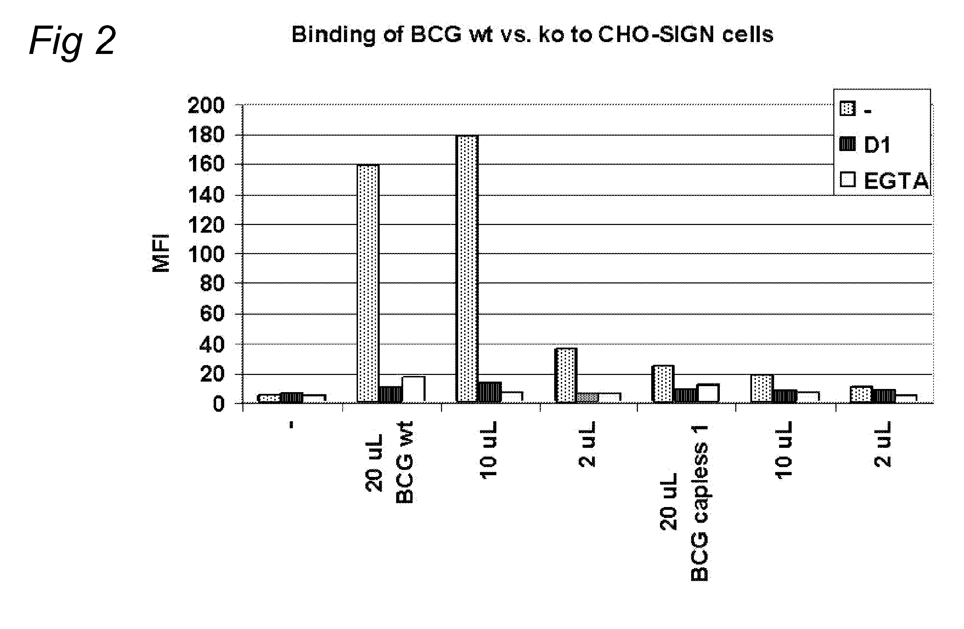

Figure 2 The binding of wild type *M. bovis* BCG and a *M*. *bovis* BCG capless 2 knock-out mutant (in which the homologue of Rc1653c has been disrupted) to CHO-cells expressing DC-SIGN. D1 is an inhibitory monoclonal directed against DC-SIGN, addition of which abolishes DC-SIGN specific binding. Addition of the calcium-gelator EGTA demonstrates the involvement of a C-type lectin.

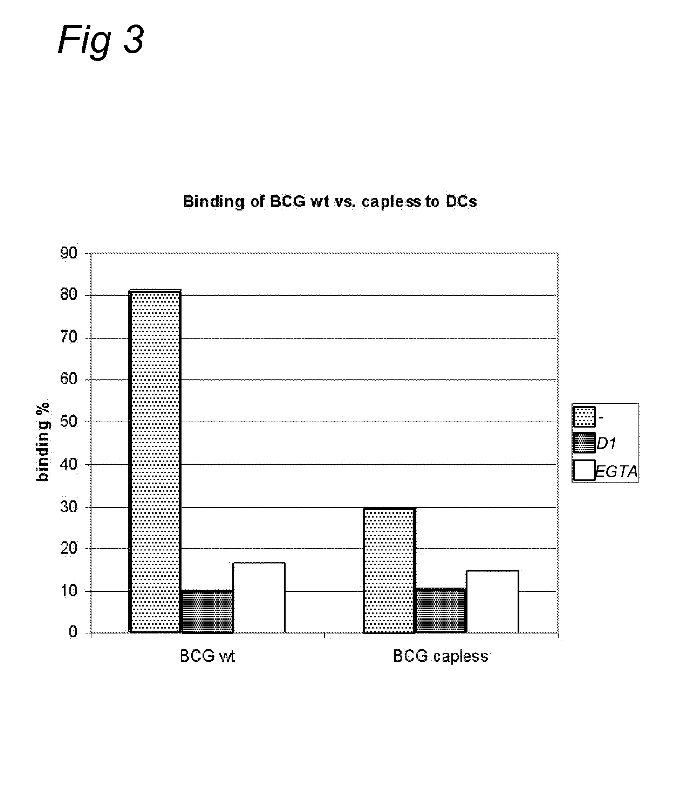

Figure 3 The binding of wild type *M. bovis* BCG and a *M*. *bovis* BCG capless 2 knock-out mutant (in which the homologue of Rc1653c has been disrupted) to isolated dendritic cells (DCs). D1 and EGTA are as described in Figure 2.

### Examples

### Example 1: Characterization of a monoclonal antibody specific for the manLAM mannose cap

To allow characterization of anti-LAM monoclonal antibodies, synthetic oligosaccharides (including (man)₁-ara, (man)₂-ara, and (man)₃-ara) representing the non-reducing terminus of manLAM were prepared (2) and coupled to a protein (bovine serum albumin, BSA) or polyacrylamide carrier (14). These neoglycoconjugates were used tot screen a large library (n>200) of monoclonal antibodies directed to *M*. *paratuberculosis* (Mabs were made available by P.T.J. Willemsen, Research Institute of Animal Husbandry, Lelystad). Cap specific Mabs (56.49.1A and 55.92.1A1) were thus obtained. Additional Elisa tests showed that these Mabs react with manLAM but not with araLAM. These Mabs are thus specific for the mannose cap and have the ability to detect mannose caps in dot-blot immunoassays. In this type of assay mycobacteria (*M. marinum*-a close relative of *M. tuberculosis,* and *M. smegmatis*) are spotted onto nitrocellulose membranes, baked, washed, incubated with Mab, washed, incubated with conjugate (goat-anti-mouse IgM-peroxydase) and immunostained. As expected only spots of *M. marinum* (expressing manLAM) were stained and not the spots of *M. smegmatis* (expressing araLAM). Further confirmation that these Mabs detect only the mannose cap was obtained by Western blotting, where it was found that the Moabs react with a heterogeneous band of the MW expected for manLAM. These Mabs were subsequently used to screen a transposon library of *M*. *marinum* strain E 11.

### Example 2: Screening a M. marinum transposon library with anti-cap Moabs

The mycobacteriophage mycomarT7 was obtained from Dr. E.J. Rubin. This phage is non-lytic for *M. marinum* and contains a *mariner* transposon with a kanamycin cassette. Phage and bacterial cells of *M. marinum* strain E11 were incubated and plated on 7H9 plates with kanamycin (25µg/ml). Transposants were grown and transferred individually to a novel plate in a grid-like pattern and subsequently spotted onto nitrocellulose. After testing 1000 transposants a single negative colony was isolated.

### Example 3: Phenotypic characterization of the capless mutant ("capless 2")

Bacterial cells of this mutant (designated capless 2) and the E11 parent were disrupted in the beadbeater with 0.1 mm beads and subjected to SDS-PAGE, blotted and immunostained with the anti-cap Mab, as well as a Mab specific for the arabinan domain of LAM (Mab F30-6, obtained from A. Kolk, KIT Amsterdam). The anti-ara Mab stained both the capless mutant and the E11 parent whereas the anti-cap Mab only stained the E11 parent cells. In addition, gels were stained with Coomassie and these data indicated that parent and mutant have very similar overall banding patterns suggesting that no major rearrangement in the bacterial cell wall have taken place after inactivation of the gene responsible for cap synthesis. A growth curve showed that the mutant grows at approximately the same rate as the parent strain. An alternative way of investigating the presence of the mannose cap has been described in the literature and consists of chemical analysis of purified LAM (e.g. by capillary electrophoresis; 16).

### Example 4: Identifying the gene responsible for cap synthesis

DNA was isolated from the capless 2 mutant and ligation-mediated PCR (LM-PCR) was performed to identify the gene where the transposon insertion had taken place. First, genomic DNA was digested with *Sall* and subsequently, the digest was ligated with the adaptors (i.e. a partial hybrid of the following DNA primers Salgd: TAGCTTATTCCTCAAGGCACGAGC and Salpt: TCGAGCTGTGC); finally, PCR was performed with a primer inside the transposon (MycoMarT7 pr-1: CCCGAAAAGTGCCACCTAAATTGTAAGCG or MycoMarT7 pr-2: CGCTTCCTCGTGCTTTACGGTATCG); the other primer was Salgd. Indeed a PCR product was formed. Subsequently, the PCR product was sequenced in an ABI sequencer. The obtained DNAsequence was Blasted (BLASTN) at NCBI. Hits were found for the mycomarT7 *mariner* transposon, ending in TA as expected for transposants with this mariner. Hence, the *mariner* sequence was removed from the ABI sequence and the "cleaned" data was Blasted against the contigs of *M. marinum* at the Sanger website (no ORF numbers are available at the time of writing) (http://www.sanger.ac.uk/Projects/M_marinum/). The sequence was found to be identical to a part of the *M. marinum* sequence identified. A DNA sequence was put together consisting of the *M. marinum* sequence identified (SEQ ID NO. 7) plus 1000 bp upstream from the 5'end and downstream from the 3'end. This sequence was Blasted (tblastx ) against *M. tuberculosis* Rv37. The homologue of the putative mannosyltransferase was Rv1635c. Blasting of the gene showed (Table 1) that orthologues were present in the second sequenced strain of *M. tuberculosis* CDC 1551 (MT1671); *M. paratuberculosis*/*avium* (ORFS MAP 1338c and 3805c), *M. bovis* (MB1661c) and *M. leprae* (ML1389); no significant hits were found in the genome of *M. smegmatis.* As Table 1 shows, identities at the amino acid level were between 70% (with the *M. tuberculosis* homologs) and 42% (with MAP3805c).

Likwise, via TBLASTX at http://www.sanger.ac.uk/cgi-bin/blact/submitblast/m_bovis in *M. bovis* BCG (consisting of assembled contigs, no completed full genome sequence available yet and no ORF numbers assigned yet) an ORF was identified with high identity (>70%) to the *M .marinum* sequence. On the DNA level, the sequence of the *M. bovis* BCG homologue was virtually identical (>98%) to that of *M*. *bovis* wildtype (SEQ ID NO. 8).

**Table 1. Amino acid sequence identities and similarities between the M. marinum cap- specific mannosyltransferase and orthologs.**

| Species | Protein | SEQ ID NO. | Identity % | Similarity % |
|---|---|---|---|---|
| *M.tuberculosis* | Rv1635c | 1 | 70 | 77 |
| *M.tuberculosis* | MT1671 | 2 | 70 | 77 |
| *M. bovis* | MB1661c | 3 | 70 | 77 |
| *M.paratuberculosis* | MAP1338c | 4 | 63 | 73 |
| *M.leprae* | ML1389 | 5 | 63 | 73 |
| *M.paratuberculosis* | MAP3805c | 6 | 42 | 58 |
| *Streptomyces* | SAV5089 | - | 22 | 35 |

### Example 5: Cap-expressing complementants of capless 2 and M. smegmatis

The capless 2 gene was amplified (using genomic DNA of *M. marinum*) with primers TTGGAATTTCAAGCAGCACA and ACATTGCAGTTGGTCTCG and Expand polymerase. The PCR product was cloned into *Smal*-digested pUC18, cut out with *Pstl* and *EcoRV* and cloned into shuttle vector pSMT3-eGFP (digested with *Pstl* and *EcoRV*), and electroporated into capless 2 and *M. smegmatis* mc²155. SDS-PAGE-immunoblots (Figure 1) indeed showed that complementation restored cap synthesis in capless 2 and moreover, also induced cap expression in *M. smegmatis*; these data demonstrate that the Rv1635c homologue of *M. marinum* is able to restore cap synthesis both in the *M. marinum* capless 2 mutant and *M. smegmatis.* We therefore conclude that this gene is both necessary and sufficient for cap synthesis in mycobacteria.

### Example 6: Construction of an unmarked capless M. bovis BCG

Using the pNIL/pGOAL-procedure developed by Parish and Stoker (17), an unmarked knock-out mutant of the Rc1653c homologue in *M. bovis* BCG was prepared. Bacterial cells of this BCGcapless mutant and the BCG parent were disrupted in the beadbeater with 0.1 mm beads and subjected to SDS-PAGE, blotted and immunostained with the anti-cap Mab, as well as a Mab specific for the arabinan domain of LAM as described in Example 3. The anti-ara Mab stained both the BCGcapless mutant and the BCG parent whereas the anti-cap Mab only stained the BCG parent cells (data not shown). In addition, gels were stained with Coomassie and these data indicated that parent and mutant have very similar overall banding patterns suggesting that no major rearrangement in the bacterial cell wall have taken place after inactivation of the gene responsible for cap synthesis (data not shown).

A growth curve showed that the mutant grows at approximately the same rate as the parent strain from which we conclude that an intact Rc1653c homologue in *M*. *bovis* BCG is not essential for growth *in vitro* (data not shown).

### Example 7: The capless M. bovis BCG mutant does not bind to DC-SIGN

Binding of mycobacteria to eukaryotic cells was evaluated as described in ref. 4. In short, bacteria were grown, washed and labelled with FICT. Cells that express DC-SIGN on their surface were: human dendritic cells, K562 and CHO-cells transfected with a DC-SIGN DNA construct; the isolation of these cells was desribed before (Ref. 4). Bacteria and eukaryotic cells were mixed (in varying proportions) and binding evaluated by FACS analysis and data expressed as MFI (mean fluorescence intensity). As control for the specificity of binding to DC-SIGN, an inhibitory monoclonal directed against DC-SIGN (D1) was included, or the calcium-gelator EGTA was added to demonstrate the involvement of a C-type lectin. As shown in Figures 2 and 3, as compared to parent strain the capless BCG binds much less to either transfected cells and to human DC as compared to wild type BCG. Together these data prove that the interaction between the mannose cap of LAM and DC-SIGN determine the interaction between mycobacteria and human DC, a prerequisite for a changed immunological signalling.

### References

1. Briken, V., S. A. Porcelli, G. S. Besra, and L. Kremer. 2004. Mycobacterial lipoarabinomannan and related lipoglycans: from biogenesis to modulation of the immune response. Mol Microbiol 53:391-403.
2. Gadikota RR, Callam CS, Appelmelk BJ, and Lowary TL. 2003. Synthesis of oligosaccharide fragments of mannosylated lipoarabinomannan appropriately functionalized for neoglycoconjugate preparation. J Carbohydr Chem 22:459-480.
3. Gagliardi, M. C., R. Teloni, S. Mariotti, E. Iona, M. Pardini, L. Fattorini, G. Orefici, and R. Nisini. 2004. Bacillus Calmette-Guerin shares with virulent Mycobacterium tuberculosis the capacity to subvert monocyte differentiation into dendritic cell: implication for its efficacy as a vaccine preventing tuberculosis. Vaccine 22:3848-3857.
4. Geijtenbeek, T. B., S. J. Van Vliet, E. A. Koppel, M. Sanchez-Hernandez, C. M. Vandenbroucke-Grauls, B. Appelmelk, and Y. Van Kooyk. 2003. Mycobacteria target DC-SIGN to suppress dendritic cell function. J Exp Med 197:7-17.
5. Guy, M. R., P. A. Illarionov, S. S. Gurcha, L. G. Dover, K. J. Gibson, P. W. Smith, D. E. Minnikin, and G. S. Besra. 2004. Novel prenyl-linked benzophenone substrate analogues of mycobacterial mannosyltransferases. Biochem. J 382:905-912.
6. Martino, A., A. Sacchi, N. Sanarico, F. Spadaro, C. Ramoni, A. Ciaramella, L. P. Pucillo, V. Colizzi, and S. Vendetti. 2004. Dendritic cells derived from BCG-infected precursors induce Th2-like immune response. J Leukoc. Biol. 76:827-34.
7. Mollenkopf, H. J., M. Kursar, and S. H. Kaufmann. 2004. Immune response to postprimary tuberculosis in mice: Mycobacterium tuberculosis and Mycobacterium bovis bacille Calmette-Guerin induce equal protection. J Infect. Dis. 190:588-597.
8. Pathak, A. K., V. Pathak, J. M. Riordan, S. S. Gurcha, G. S. Besra, and R. C. Reynolds. 2004. Synthesis of mannopyranose disaccharides as photoaffinity probes for mannosyltransferases in Mycobacterium tuberculosis. Carbohydr. Res 339:683-691.
9. Rook, G. A., R. Hernandez-Pando, K. Dheda, and S. G. Teng. 2004. IL-4 in tuberculosis: implications for vaccine design. Trends Immunol. 25:483-488.
10. Sassetti, C. M., D. H. Boyd, and E. J. Rubin. 2001. Comprehensive identification of conditionally essential genes in mycobacteria. Proc Natl Acad Sci U S A 98:12712-12717.
11. Tailleux, L., O. Schwartz, J. L. Herrmann, E. Pivert, M. Jackson, A. Amara, L. Legres, D. Dreher, L. P. Nicod, J. C. Gluckman, P. H. Lagrange, B. Gicquel, and O. Neyrolles. 2003. DC-SIGN is the major Mycobacterium tuberculosis receptor on human dendritic cells. J Exp Med 197:121-127.
12. Vergne I, Chua J, Singh SB, Deretic V. 2004. Cell biology of mycobacterium tuberculosis phagosome. Annu Rev Cell Dev Biol.;20:367-94.
13. Hmama Z, Sendide K, Talal A, Garcia R, Dobos K, Reiner NE. 2004. Quantitative analysis of phagolysosome fusion in intact cells: inhibition by mycobacterial lipoarabinomannan and rescue by an 1 alpha,25-dihydroxyvitamin D3-phosphoinositide 3-kinase pathway. J Cell Sci. 117:2131-40.
14. Bovin NV, Korchagina EYu, Zemlyanukhina TV, Byramova NE, Galanina OE, Zemlyakov AE, Ivanov AE, Zubov VP, Mochalova LV. 1993. Synthesis of polymeric neoglycoconjugates based on N-substituted polyacrylamides. Glycoconj J. 10:142-51.
15. Gordon S. 2003. Alternative activation of macrophages. Nat Rev Immunol. 3:23-35.
16. Nigou J, Vercellone A, Puzo G. 2000. New structural insights into the molecular deciphering of mycobacterial lipoglycan binding to C-type lectins: lipoarabinomannan glycoform characterization and quantification by capillary electrophoresis at the subnanomole level. J Mol Biol. 299:1353-62.
17. Parish T, Stoker NG. 2000. Use of a flexible cassette method to generate a double unmarked Mycobacterium tuberculosis tlyA plcABC mutant by gene replacement. Microbiology. 146: 1969-75.

## Claims

1. A mycobacterial vector comprising a nucleotide sequence encoding a polypeptide having mycobacterial manLAM cap-specific mannosyl transferase activity, wherein the polypeptide has at least 35% amino acid identity with at least one of SEQ ID NO. 1 - 6.

2. A mycobacterial vector according to claim 1, wherein the nucleotide sequence encoding the polypeptide is operably linked to a promoter.

3. A mycobacterial vector according to claim 2, wherein the promoter is heterologous to the nucleotide sequence encoding the polypeptide.

4. A host cell comprising a vector as defined in any one of claims 1 - 3.

5. A host cell according to claim 4, wherein the host cell is a mycobacterial cell.

6. A host cell according to claim 5, wherein the cell is a cell of a slow growing virulent *Mycobacterium.*

7. A host cell according to claim 6, wherein the *Mycobacterium* is selected from *M. bovis, M. tuberculosis, M. avium, M. paratuberculosis, M. leprae, M. ulcerans* and *M. marimum.*

8. A host cell according to claims 6 or 7, wherein the *Mycobacterium* is attenuated.

9. A host cell according to claim 8, wherein the *Mycobacterium* is the vaccine strain *M. bovis* bacillus Calmette-Guérin.

10. A pharmaceutical composition comprising a host cell as defined in any one of claims 4 - 9, and a pharmaceutically acceptable carrier.

11. A pharmaceutical composition according to claim 10, further comprising an adjuvant.

12. A host cell as defined in any one of claims 4 - 9, for use as a medicament.

13. Use of host cell as defined in any one of claims 4 - 9, for the manufacture of a medicament for the treatment or prophylaxis of a mycobacterial infection.

14. A host cell as defined in any one of claims 4 - 9, for the treatment or prophylaxis of a mycobacterial infection.

15. A method for identification of a compound that inhibits a mycobacterial manLAM cap-specific mannosyl transferase wherein the method comprises the steps of
(a) contacting the compound with a polypeptide as defined in claim 1, or with a host cell as defined in any one of claims 4 - 9, that expresses the polypeptide; and,
(b) determining the manLAM cap-specific mannosyl transferase activity of the polypeptide.
